(19)

(11) EP 1 866 070 B1

(12) EUROPÄISCHE PATENTSCHRIFT

(45) Veröffentlichungstag und Bekanntmachung des Hinweises auf die Patenterteilung:
**11.12.2013 Patentblatt 2013/50**

(21) Anmeldenummer: **06708446.7**

(22) Anmeldetag: **22.02.2006**

(51) Int Cl.:
***B01J 8/22*** *(2006.01)* ***G01N 21/00*** *(2006.01)*
***C07C 209/36*** *(2006.01)* ***C07C 211/50*** *(2006.01)*
***B01J 8/00*** *(2006.01)*

(86) Internationale Anmeldenummer:
**PCT/EP2006/060174**

(87) Internationale Veröffentlichungsnummer:
**WO 2006/089906 (31.08.2006 Gazette 2006/35)**

# (54) VERFAHREN UND VORRICHTUNG ZUR HERSTELLUNG VON AROMATISCHEN AMINEN ODER ALIPHATISCHEN AMINOALKOHOLEN

METHOD AND APPARATUS FOR PRODUCING AROMATIC AMINES OR ALIPHATIC AMINO ALCOHOLS

PROCEDE ET APPAREILLAGE DE PRODUCTION D’AMINES AROMATIQUES OU D’AMINO-ALCOOLS ALIPHATIQUES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **23.02.2005 DE 102005008613**

(43) Veröffentlichungstag der Anmeldung:
**19.12.2007 Patentblatt 2007/51**

(73) Patentinhaber: **BASF SE**
**67056 Ludwigshafen (DE)**

(72) Erfinder:
- **VANOPPEN, Dominic**
  **67135 Schifferstadt (DE)**
- **VAN LAAR, Frederik**
  **67117 Limburgerhof (DE)**
- **BEUERMANN, Thomas**
  **68161 Mannheim (DE)**
- **KRUG, Georg**
  **69509 Mörlenbach (DE)**
- **OEHLENSCHLÄGER, Steffen**
  **67063 Ludwigshafen (DE)**
- **SCHWAB, Ekkehard**
  **67434 Neustadt (DE)**
- **VOSS, Hartwig**
  **67227 Frankenthal (DE)**
- **MORGENSCHWEIS, Konrad**
  **01108 Dresden (DE)**
- **PENZEL, Ulrich**
  **01945 Tettau (DE)**
- **TITTELBACH-HELMRICH, Dietrich**
  **01561 Tauscha (DE)**

(56) Entgegenhaltungen:
**EP-A- 0 971 225 WO-A-02/088860**

- **PATENT ABSTRACTS OF JAPAN Bd. 2003, Nr. 12, 5. Dezember 2003 (2003-12-05) & JP 2003 261530 A (NIPPON SODA CO LTD), 19. September 2003 (2003-09-19)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 866 070 B1

Printed by Jouve, 75001 PARIS (FR)

## Beschreibung

Die Erfindung betrifft ein Verfahren zur Herstellung von aromatischen Aminen durch Hydrierung von Nitroaromaten oder von aliphatischen Aminoalkoholen durch Hydrierung von Nitroalkoholen in Gegenwart von Katalysatoren.

Die Herstellung von Aminen, insbesondere von aromatischen Mono- und/oder Polyaminen durch katalytische Hydrierung von Mono- und/oder Polynitroverbindungen ist im Stand der Technik bekannt.

DE-OS 2 044 657 hat zum Beispiel ein Verfahren zur Herstellung von Toluylendiamin durch Hydrieren von Dinitrotoluol in Gegenwart von Nickel oder Ruthenium enthaltenden Hydrierkatalysatoren zum Gegenstand.

EP-B1 1 138 665 bezieht sich auf ein Verfahren zur katalytischen Hydrierung von aromatischen Nitroverbindungen, bei dem die Hydrierung kontinuierlich mit einem Katalysator realisiert wird, der mindestens Nickel und gegebenenfalls Aluminium umfasst. Nach der Durchführung der Hydrierung wird der Katalysator in einer Trennzone von der Reaktionsmischung getrennt.

Das Dokument EP-B1 0 978 505 beschreibt ein Verfahren zum Hydrieren einer nitroaromatischen Zusammensetzung durch den Kontakt der nitroaromatischen Zusammensetzung mit Wasserstoff in einem Reaktor unter Verwendung eines monolithischen Katalysators. Nach der Durchführung der Hydrierung erfolgt ein kontinuierliches Entfernen des hydrierten Reaktionsproduktes, das nicht umgesetztes Dinitrotoluol, Wasser und Toluoldiamin umfasst, aus dem Reaktor. Weitere Dokumente aus dem Stand der Technik, die Verfahren zur Herstellung von Aminen durch Hydrierung von Nitroverbindungen beschreiben, sind zum Beispiel EP-A 634 391 oder WO 00/35852.

Das bei der Durchführung der Hydrierung von Nitroaromaten in einem Reaktor erhaltene Reaktionsgemisch enthält außer den aromatischen Aminen auch Nitro- und Nitrosoverbindungen, die zum Beispiel die als Edukte eingesetzten Nitroaromaten oder in dem Reaktor gebildete Zwischenprodukte umfassen. Nitro- und Nitrosoverbindungen können sich insbesondere beim Erhitzen explosionsartig zersetzen. Aus Sicherheitsgründen ist daher eine Überwachung des Reaktionsgemisches in Bezug auf die darin vorhandene Konzentration an Nitro- und Nitrosoverbindungen wichtig. Das Sicherheitsrisiko steigt in dem Maße an, in dem die Reaktorgröße zunimmt und die Verweilzeiten in dem Reaktor reduziert werden. Es muss sichergestellt werden, dass diese explosiven Verbindungen vollständig im Reaktor umgesetzt werden, bevor das Reaktionsgemisch zum Beispiel einer nachfolgenden Destillation zugeführt wird.

Des Weiteren ergibt sich bei der katalytischen Hydrierung von Nitroaromaten das Problem, dass die Katalysatoren mit der Zeit deaktiviert werden. Je geringer die Aktivität der Katalysatoren ist, um so geringer ist der zu Aminen umgesetzte Anteil der Edukte, so dass der in dem Reaktor verbleibende Anteil an nicht umgesetzten Nitroaromaten steigt. Daher ist eine Überwachung der Katalysatoraktivität notwendig, insbesondere um dem Reaktor eine ausreichende Menge an unverbrauchtem Katalysator zuzuführen.

Im Stand der Technik wird die Konzentration der Nitro- und Nitrosoverbindungen und die Aktivität des Katalysators anhand von Gaschromatographie-Proben verfolgt (zum Beispiel in dem Verfahren gemäß WO 03/066571 A1).

WO 02/088860 A2 offenbart eine Vorrichtung zur Steuerung von chemischen Syntheseprozessen, mit einer Probenentnahmevorrichtung, einer Aufbereitungseinrichtung, einer Einrichtung zur Konzentrationsbestimmung wenigstens einer Substanz in der Probe, eine Auswertungseinrichtung und einer Koordinierungseinrichtung.

Die Aufgabe der Erfindung war es, ein Verfahren zur Herstellung von aromatischen Aminen durch Hydrierung von Nitroaromaten in Gegenwart von Katalysatoren in einem Reaktor bereitzustellen, das eine einfache Online-Überwachung der Konzentration von Nitro- und Nitrosoverbindungen in einem in dem Reaktor enthaltenen Reaktionsgemisch erlaubt. Die Aufgabe bestand ferner darin, eine Online-Überwachung der Katalysatoraktivität in dem Reaktor zu ermöglichen.

Diese Aufgabe wird erfindungsgemäß gelöst durch ein Verfahren zur Herstellung von aromatischen Aminen durch Hydrierung von Nitroaromaten oder von aliphatischen Aminoalkoholen durch Hydrierung von Nitroalkoholen in Gegenwart von Katalysatoren, wobei in einem Reaktor ein fluides, Amine oder Aminoalkohole enthaltendes Reaktionsgemisch entsteht, aus dem die Katalysatoren abgetrennt werden. Nach dem Abtrennen der Katalysatoren wird eine Messung der Absorption von UV-VIS-Strahlung durch das Reaktionsgemisch zur Konzentrationsbestimmung von Nitro- und Nitrosoverbindungen in dem Reaktionsgemisch durchgeführt.

Diese Aufgabe wird weiterhin gelöst durch eine Vorrichtung mit den Merkmalen des Anspruchs 10.

Unter Nitroverbindungen werden in diesem Zusammenhang organische Verbindungen verstanden, in denen ein Wasserstoffatom durch eine Nitrogruppe ($NO_2$-Gruppe) ersetzt ist. Unter Nitrosoverbindungen sind organische Verbindungen zu verstehen, die die Nitrosogruppe (NO-Gruppe) an ein aromatisches Kohlenstoffatom gebunden enthalten. Unter Aminen sind Mono-, Di- und Polyamine zu verstehen. Das fluide Reaktionsgemisch kann sowohl flüssig als auch gasförmig sein.

UV-VIS-Strahlung ist elektromagnetische Strahlung im sichtbaren oder im UV-Bereich. Aus einer gemessenen Absorption von UV-VIS-Strahlung durch das Reaktionsgemisch lässt sich die Konzentration von Nitro- und Nitrosoverbindungen in dem Reaktionsgemisch bestimmen. Wenn monochromatische UV-VIS-Strahlung mit einer Anfangsintensität $I_0$ eine verdünnte Lösung eines absorbierenden Stoffes (zum Beispiel das Reaktionsgemisch) der Dicke d durchdringt, so beschreibt das Lambert-Beer'sche Gesetz die Absorption der Strahlung durch die Lösung. Das Lambert-Beer'sche Gesetz lautet:

$$2{,}3 \bullet \log \frac{I_0}{I} = E = \varepsilon \bullet d \bullet c$$

wobei

$I_0$: Intensität der Strahlung vor Eintritt in die Lösung

I: Intensität der Strahlung nach Durchdringen der Schichtdicke d

d: Schichtdicke (zum Beispiel Küvettenmaß) des Fluides (z.B. der Lösung) in cm

c: Konzentration der absorbierenden Substanz in mol/l

ε: molarer Extinktionskoeffizient in l/(mol x cm) (Stoffkonstante)

E: Extinktion.

Es ergibt sich ein linearer Zusammenhang zwischen der Konzentration des Fluides und der Extinktion. Dadurch ist die Bestimmung der Konzentration einer absorbierenden Probe durch die Messung der Extinktion (zum Beispiel mit einem Spektralphotometer) mittels einer Eichkurve oder bekannten Extinktionskoeffizienten möglich (Photometrie).

Die Abtrennung der Katalysatoren aus dem Reaktionsgemisch vor der Absorptionsbestimmung vereinfacht bei dem erfindungsgemäßen Verfahren die Aufarbeitung des Endprodukts. Ferner muss das Reaktionsgemisch zur Absorptionsmessung weitgehend von den schwarzen Katalysatorsteilchen befreit sein, da diese die UV-VIS-Spektroskopie stören.

Zur Konzentrationsbestimmung der Nitro- und Nitrosoverbindungen kann ein Absorptionsspektrum in einem Wellenlängenbereich der UV-VIS-Strahlung oder die Absorption (Extinktion) von UV-VIS-Strahlung mit einer Wellenlänge gemessen werden. Vorzugsweise erfolgt die Messung monochromatisch (mit Strahlung einer ausgewählten Wellenlänge). Dies ist vollkommen ausreichend, um die Konzentration der Nitro- und Nitrosoverbindungen in dem Reaktionsgemisch zu bestimmen. Die Wellenlänge der Strahlung wird so ausgewählt, dass Beiträge anderer Komponenten des Reaktionsgemisches, insbesondere der Amine zu der Absorption der Strahlung möglichst gering sind.

Die Messung des Absorptionsspektrums in einem Wellenlängenbereich der UV-VIS-Strahlung hat den Vorteil, dass fehlerhafte Messungen, die zum Beispiel durch Gasblasen in dem Reaktionsgemisch verursacht werden können, leicht erkannt und nicht zur Konzentrationsbestimmung herangezogen werden. Eine solche Messung eines Absorptionsspektrums erfordert jedoch ein aufwendigeres Photometer, bei dem sowohl die darin enthaltene Lichtquelle, als auch der Detektor einen solchen Wellenlängenbereich abdecken müssen.

Gemäß einer bevorzugten Ausführungsform der vorliegenden Erfindung wird zusätzlich eine Messung der Absorption von UV-VIS-Strahlung in einem anderen Wellenlängenbereich oder bei einer anderen Wellenlänge durch das Reaktionsgemisch zur Nulllinienkorrektur durchgeführt. Eine solche Nulllinienkorrektur ist notwendig, um den Einfluss von Intensitätsschwankungen der UV-VIS-Strahlung emittierenden Lichtquelle auszugleichen. Der Wellenlängenbereich oder die Wellenlänge werden dafür so ausgewählt, dass die Nitro- und Nitrosoverbindungen in diesem Wellenlängenbereich beziehungsweise bei dieser Wellenlänge keinen oder einen vernachlässigbar kleinen Beitrag an der gemessenen Absorption durch das Reaktionsgemisch haben. Diese Messung ergibt einen Korrekturwert. Die Konzentrationsbestimmung der Nitro- und Nitrosoverbindungen erfolgt in einem Wellenlängenbereich oder bei einer Wellenlänge, bei dem/bei der im Wesentlichen die Nitro- und Nitrosoverbindungen in dem Reaktionsgemisch die UV-VIS-Strahlung absorbieren. Die gemessene Absorption wird dann um den Korrekturwert korrigiert, der in dem anderen Wellenlängenbereich/bei der anderen Wellenlänge zur Nulllinienkorrektur gemessen wurde. Die Messung der Absorption durch die Nitro- und Nitrosoverbindungen und der Korrekturwerte kann gleichzeitig durch zwei auf verschiedene Wellenlängenbereiche) eingestellte Photometer kontinuierlich erfolgen. Es kann aber auch mit einem Photometer ein beide Wellenlängenbereiche) umfassendes Spektrum aufgenommen werden oder es können abwechselnd Messungen bei den zwei verschiedenen Wellenlängen durchgeführt werden.

Die Katalysatoren werden bei dem Verfahren der vorliegenden Erfindung aus dem Reaktionsgemisch mit mindestens einem Abtrennverfahren abgetrennt, das aus der Gruppe Membranfiltration, Sedimentation und Zentrifugation ausgewählt ist oder mit einem beliebigen anderen, im Stand der Technik bekannten Verfahren. Das Abtrennen eines in einem Reaktionsgemisch enthaltenen Katalysators ist zum Beispiel aus DE-A1 32 45 318, DE-A1 30 40 631 oder WO 03/066571 bekannt. Es ist auch eine Kombination von Abtrennverfahren möglich (zum Beispiel Sedimentation und anschließende Membranfiltration), um eine möglichst vollständige Abtrennung des Katalysators aus dem Reaktionsgemisch zu erreichen, bevor Absorptionsmessungen durchgeführt werden.

Bei der Vorrichtung der vorliegenden Erfindung erfolgt die Abtrennung der Katalysatoren aus dem Reaktionsgemisch mittels Membranfiltration.

Die Messung der Absorption von UV-VIS-Strahlung kann bei der vorliegenden Erfindung bei einem gegenüber dem Umgebungsdruck erhöhten Druck und/ oder bei Umgebungsdruck durchgeführt werden. Beispielsweise kann ein Photometer in einem Bereich hinter einem Membranfilter zur Abtrennung des Katalysators angeordnet sein, in dem das aus dem Filter austretende Filtrat unter einem gegenüber dem Umgebungsdruck erhöhten Druck steht. Die Absorptionsmessung kann aber auch in einem Bereich einer Vorrichtung zur Herstellung von aromatischen Aminen angeordnet sein, in dem der Katalysator bereits abgetrennt ist und das Reaktionsgemisch auf Umgebungsdruck entspannt ist.

Die Messung der Absorption von UV-VIS-Strahlung durch das Reaktionsgemisch kann bei der vorliegenden Erfindung in dem aus dem Reaktor entnommenen Hauptstrom oder bevorzugt in einem von dem Hauptstrom abzweigenden Nebenstrom erfolgen.

Gemäß einer bevorzugten Ausführungsform der vorliegenden Erfindung wird in Abhängigkeit von der Konzentrationsbestimmung der Nitro- und/oder Nitrosoverbindungen in dem Reaktionsgemisch eine dem Reaktor zugeführte Menge an Nitroaromaten, Nitroalkoholen oder Katalysatoren geregelt. Gegenstand der vorliegenden Erfindung ist daher auch ein Regelungsverfahren, das bei der Herstellung von aromatischen Aminen durch Hydrierung von Nitroaromaten oder von aliphatischen Aminoalkoholen durch Hydrierung von Nitroalkoholen in Gegenwart von Katalysatoren in einem Reaktor in Abhängigkeit von einer per Absorptionsmessung bestimmten Konzentration an Nitro- und/oder Nitrosoverbindungen im Reaktionsgemisch die Menge an Katalysator und/oder Nitroaromaten oder Nitroakoholen regelt, die dem Reaktor zugeführt wird. Die gemessene Konzentration an Nitro- und/oder Nitrosoverbindungen in dem Reaktionsgemisch steigt, wenn die Aktivität des in dem Reaktor vorhandenen Katalysators abnimmt. In diesem Fall kann einerseits die Menge an zugegebenen Nitroaromaten oder Nitroalkoholen reduziert werden oder die zugegebene Menge an neuem aktivem Katalysator erhöht werden.

Vorzugsweise werden mit dem erfindungsgemäßen Herstellungsverfahren aromatische Amine durch Hydrierung von Nitroaromaten mit einer oder mehreren Nitrogruppen und 6 bis 18 C-Atomen hergestellt. Die Nitroaromaten sind beispielsweise Nitrobenzol, Nitrobenzole wie 1,2-, 1,3-, 1,4-Dinitrobenzol, Nitrotoluole, wie o-, m-, p-Nitrotoluol, Dinitrotoluole wie 2,4-, 2,6-, 2,3-, 3,4-, 2,5-Dinitrotoluol, 2,4,6-Trinitrotoluol, Nitroxylole, wie 1,2-Dimethyl-3-, 1,2-Dimethyl-4-, 1,4-Dimethyl-2-, 1,3-Dimethyl-2-, 2,4-Dimethyl-1- und 1,3-Dimethyl-5-nitrobenzol, Nitronaphthaline, wie 1-, 2-Nitronaphthalin, 1,5- und 1,8-Dinitronaphthalin, Chlornitrobenzole, wie 2-Chlor-1,3-, 1-Chlor-2,4-dinitrobenzol, o-, m-, p-Chlornitrobenzol, 1,2-Dichlor-4-, 1,4-Dichlor-2-, 2,4-Dichlor-1- und 1,2-Dichlor-3-nitrobenzol, Chlornitrotoluole, wie 4-Chlor-2-, 4-Chlor-3-, 2-Chlor-4- und 2-Chlor-6-nitrotoluol, Nitroaniline, wie o-, m-, p-Nitroanilin sowie beliebige Gemische aus 2 oder mehreren der genannten Nitroverbindungen. Außerdem können mit dem erfindungsgemäßen Verfahren aliphatische Aminoalkohole durch Hydrierung von Nitroalkoholen hergestellt werden. Die Nitroalkohole sind beispielsweise Tri(hydroxymethyl)nitro-methan, 2-Nitro-2-methyl-, 2-Nitro-2-ethyl-1,3-propandiol, 2-Nitro-1-butanol und 2-Nitro-2-methyl-1-propanol sowie beliebige Gemische aus 2 oder mehreren der genannten Nitroverbindungen.

Bevorzugt wird nach dem erfindungsgemäßen Verfahren Dinitrotoluol, insbesondere 2,4-Dinitrotoluol oder dessen technische Gemische mit 2,6-Dinitrotoluol zu dem entsprechenden Amin hydriert. Gemäß einer besonders bevorzugten Ausführungsform der vorliegenden Erfindung wird Toluoldiamin durch Hydrierung von Dinitrotoluol hergestellt und eine Messung der Absorption von UV-VIS-Strahlung zur Konzentrationsbestimmung im Wesentlichen von in dem Reaktionsgemisch enthaltenem Dinitrotoluol und Aminonitrotoluol durchgeführt. Aminonitrotoluol ist ein bei der Hydrierung von Dinitrotoluol entstehendes Zwischenprodukt. Vorzugsweise wird die Messung der Absorption durch im Wesentlichen Dinitrotoluol und Aminonitrotoluol in dem Reaktionsgemisch bei einer Wellenlänge im Bereich von 450 bis 550 nm, vorzugsweise bei 500 nm, durchgeführt oder ein Absorptionsspektrum in einem Wellenlängenbereich zwischen 350 und 750 nm aufgenommen. In dem Bereich von 450 bis 550 nm wird die Strahlung im Wesentlichen durch das in dem Reaktionsgemisch enthaltene Dinitrotoluol und Aminonitrotoluol absorbiert. Die Absorption durch das Toluoldiamin und das in dem Reaktionsgemisch enthaltene Wasser ist in diesem Wellenlängenbereich gering. In einem Wellenlängenbereich von 650 bis 750 nm, bevorzugt bei 700 nm, können Absorptionsmessungen zur Nulllinienkorrektur durchgeführt werden. In diesem Wellenlängenbereich liegt die Absorption durch Dinitrotoluol und Aminonitrotoluol nahezu bei Null.

Die Erfindung bezieht sich weiterhin auf eine Vorrichtung mit den Merkmalen des Anspruchs 10, zur Durchführung des erfindungsgemäßen Verfahrens mit einem Reaktor zur Hydrierung von Nitroaromaten oder von Nitroalkoholen in Gegenwart von Katalysatoren, wobei ein Reaktionsgemisch entsteht, mit einem Abtrennelement zum Abtrennen der Katalysatoren aus dem Reaktionsgemisch und mit einem UV-VIS-Spektrometer zur Messung der Absorption von UV-VIS-Strahlung durch das Reaktionsgemisch zur Konzentrationsbestimmung von Nitro- und Nitrosoverbindungen in dem Reaktionsgemisch, wobei die Abtrennung der Katalysatoren aus dem Reaktionsgemisch mittels Membranfiltration erfolgt.

Als Reaktoren werden die üblichen und bekannten Hydrierreaktoren verwendet. Beispiele hierfür sind Rührkessel, Wirbelbettreaktoren, monolitische, katalytische Hydrierreaktoren, wie beispielsweise in EP-A2 1310302 beschrieben, Rohrbündelreaktoren, Blasensäulen, die Packungen enthalten können, oder Schlaufenreaktoren, wie Loop-Venturi-Reaktoren, oder Strahlschlaufenreaktoren mit innerem und äußerem Kreislauf, wie beispielsweise in WO 00/35852 oder WO 03/068724 beschrieben.

Das Abtrennelement ist ein Membranfilter. Das UV-VIS-Spektrometer enthält eine UV-VIS-Lichtquelle und einen Detektor, zwischen denen ein Behältnis angeordnet ist, durch das das Reaktionsgemisch strömen kann. Der Weg, den das von der Lichtquelle emittierte Licht bei der Absorptionsmessung durch das in dem Behältnis vorhandene Reaktionsgemisch zurücklegt, bevor es aus dem Behältnis wieder austritt und von dem Detektor detektiert wird, ist die sogenannte Schichtdicke, die vorzugsweise zwischen 0,5 und 1,5 cm liegt. Die Lichtquelle und der Detektor sind vorzugsweise in einer Durchflusszelle montiert, die mit Reaktionsgemisch führenden Nebenstromleitungen verbunden ist.

Die Lichtquelle strahlt in einem möglichst weiten Spektralbereich (Kontinuumstrahler), falls Absorptionsspektren aufgenommen werden sollen. Für die Messung der Absorption bei einer bestimmten Wellenlänge genügt auch eine Lichtquelle, die mit einem schmalen Spektralbereich strahlt, insbesondere eine monochromatische Lichtquelle. Die Lichtquelle kann auch zwei verschiedene Lampen umfassen, um ein großes Spektrum abzudecken. Die Lichtquelle umfasst vorzugsweise eine Wolframbandlampe, eine Wasserstoff- oder eine Deuteriumlampe. Ein Monochromator kann zur Zerlegung des Lichts in einzelne Wellenlängen dienen. Der Detektor kann zum Beispiel eine Vakuumphotozelle, einen Photomultiplier oder ein Feld von Silicium-Photodioden enthalten.

Das von dem Detektor detektierte Signal wird gegebenenfalls durch einen Verstärker verstärkt und in einer Auswertungseinheit (zum Beispiel einem Rechner) ausgewertet. Das Ergebnis der aus der gemessenen Absorption bestimmten Konzentration an Nitro- und Nitrosoverbindungen wird zum Beispiel auf einer Anzeige zur Information eines Anwenders angezeigt. Es wird zur Regelung des Amin-Herstellungsverfahrens herangezogen oder löst ein Alarmsignal aus, falls eine bestimmte Nitro- und Nitrosoverbindungskonzentration überschritten wird. Ferner kann die Auswerteeinheit so programmiert sein, dass diese eine aufgrund von Gasblasen im Reaktionsgemisch fehlerhafte Messung erkennt und verwirft (d.h. nicht anzeigt, nicht zur Regelung des Verfahrens verwendet oder auch sonst nicht berücksichtigt).

Mit der erfindungsgemäßen Vorrichtung ist eine automatische Online-Messung der Konzentration der Nitro- und Nitrosoverbindungen in dem Reaktionsgemisch möglich. Durch das auf der Konzentrationsmessung basierende, bereits beschriebene Regelungsverfahren kann in vorteilhafter Weise der Katalysatorverbrauch optimiert und damit reduziert werden.

Anhand des Beispiels und der Zeichnung wird die Erfindung nachstehend näher erläutert.

Es zeigt:

Figur 1 ein UV-VIS-Absorptionspektrum, das mit einer Lösung aus Aminonitrotoluol und Dinitrotoluol in einer Toluoldiamin/$H_2O$-Matrix aufgenommen wurde,

Figur 2 ein UV-VIS-Absorptionsspektrum einer Lösung von 200 ppm Aminonitrotoluol in einer Toluoldiamin/$H_2O$-Matrix,

Figur 3 ein UV-VIS-Absorptionsspektrum einer Lösung von 350 ppm Dinitrotoluol in einer Toluoldiamin/$H_2O$-Matrix,

Figur 4 ein UV-VIS-Absorptionsspektrum eines Reaktionsgemisches mit einer TDA/$H_2O$-Matrix, das von einer Gasblase beeinflusst wurde,

Figur 5 einen Vergleich von Aminonitrotoluol- und Dinitrotoluol-Absorptionsspektren mit einem von einer Gasblase beeinflussten Spektrum und

Figur 6 die Konzentrationsüberwachung eines Pilotreaktors bei der Hydrierung von Dintrotoluol durch Messung der Absorption von UV/VIS-Strahlung durch das Reaktionsgemisch.

Die Figuren werden im Rahmen von Beispiel 1 zur Hydrierung von Dinitrotoluol näher erläutert.

Beispiel 1

In einem 5-l-Pilotreaktor wurde Dinitrotoluol an einem geträgerten Nickelkatalysator mit einer mittleren Teilchengröße von 5 bis 10 $\mu$m bei einem Druck von 20 bis 25 bar und einer Temperatur von 120 bis 125°C hydriert. Die Konzentration im Wesentlichen von Dinitrotoluol und Aminonitrotoluol in dem Reaktionsgemisch wurde durch Absorptionsmessungen bestimmt. Dazu wurde direkt hinter einem Membranfilter und stromabwärts davon hinter einem Steuerventil in einem Bereich unter Umgebungsdruck jeweils eine Quarzfensterzelle eingebaut. Die Schichtdicke betrug in beiden Zellen 1 cm. Unter Verwendung von optischen Glasfaserkabeln wurden die Zellen mit einem UV/VIS-Spektrometer verbunden, das mit einem Diodenfelddetektor ausgerüstet war.

Um das Verfahren zu eichen, wurde ein geringer Strom einer Lösung mit einer bekannten Konzentration an Dinitrotoluol und Aminonitrotoluol unter Verwendung einer HPLC-Pumpe in den aus dem Reaktor strömenden Reaktionsgemischstrom gepumpt. Dabei wurde während des Betriebs der Anlage jede Minute einmal ein Absorptionsspektrum aufgenommen, das von einem Computer ausgewertet und auf einer Festplatte gespeichert wurde. Das Computerprogramm führte auch eine Nulllinienkorrektur der aufgenommenen Spektren durch.

Die Auswertung der so aufgenommenen Spektren ergab, dass 700 nm eine geeignete Wellenlänge für die Absorptionsmessung zur Nulllinienkorrektur ist. Als Wellenlänge für die Bestimmung der Nitro- und Nitrosoverbindungskonzentration wurde eine Wellenlänge von 500 nm gewählt.

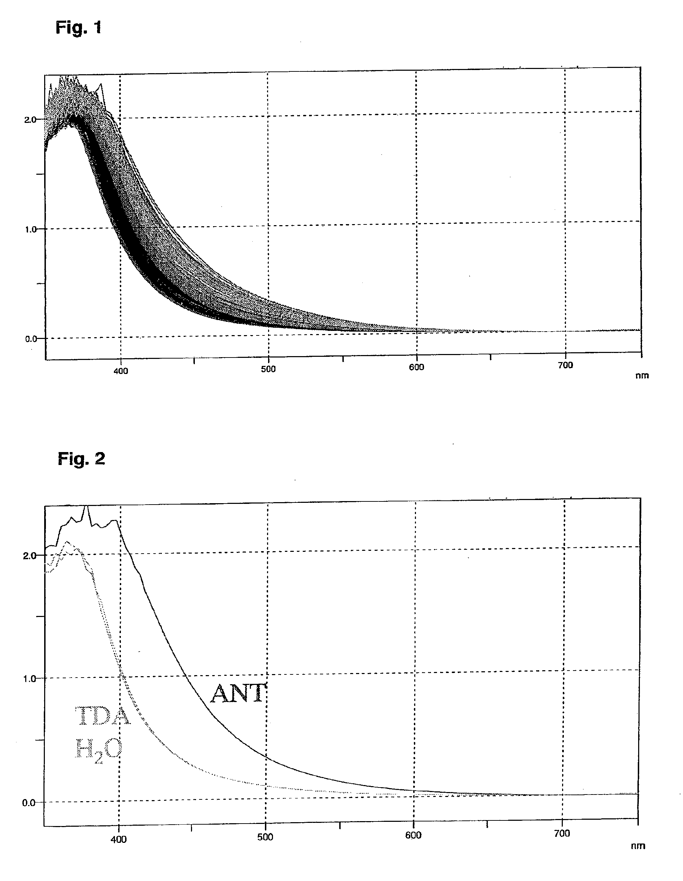

Figur 1 zeigt ein UV/VIS-Absorptionsspektrum, das mit einer Lösung aus Aminonitrotoluol und Dinitrotoluol in einer Toluoldiamin/$H_2O$-Matrix aufgenommen wurde. Die Spektren sind bereits Nulllinien-korrigiert. Auf der Y-Achse ist die Absorption (Extinktion) in Absorptionseinheiten und auf der X-Achse die Wellenlänge in nm aufgetragen. Die Spektren weisen ein Maximum bei ca. 375 nm auf. Aufgrund der Nulllinienkorrektur liegt im Bereich um 700 nm eine Absorption von Null vor. Bei 500 nm liegt die Absorption deutlich über Null. Die verschiedenen Spektren unterscheiden sich, da die untersuchten Lösungen verschiedene Nitrogruppenkonzentrationen aufwiesen.

Figur 2 zeigt ein UV/VIS-Absorptionsspektrum einer Lösung von 200 ppm Aminonitrotoluol in einer Toluoldiamin/$H_2O$-Matrix. Es handelte sich dabei um 200 ppm 4-Amino-2-nitrotoluol. Auf der Y-Achse ist wieder die Absorption (Extinktion) und auf der X-Achse die Wellenlänge aufgetragen. Eine Nulllinienkorrektur wurde durchgeführt. Die beiden mit TDA/$H_2O$ bezeichneten Kurven stellen zwei Spektren der Toluoldiamin/$H_2O$-Matrix allein dar. Die mit ANT bezeichnete Kurve ist das Absorptionsspektrum des Aminonitrotoluols in der Toluoldiamin/$H_2O$-Matrix.

Bei einer Wellenlänge von 500 nm wird der Hauptanteil des absorbierenden Lichts durch das Aminonitrotoluol absorbiert.

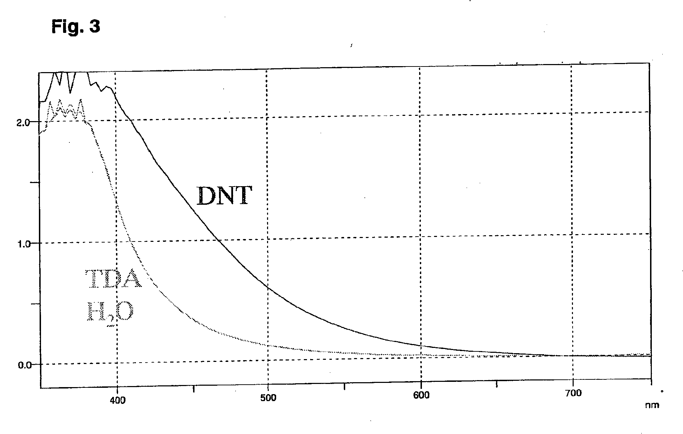

Figur 3 zeigt ein UV/VIS-Absorptionsspektrum einer Lösung von 350 ppm Dinitrotoluol in einer Toluoldiamin/$H_2O$-Matrix. Auf der Y-Achse ist die Absorption (Extinktion) und auf der X-Achse die Wellenlänge der UV/VIS-Strahlung aufgetragen. Eine Nulllinienkorrektur wurde durchgeführt. Die beiden mit TDA/$H_2O$ bezeichneten Kurven stellen wiederum zwei Spektren nur der Toluoldiamin/$H_2O$-Matrix dar. Die mit DNT bezeichnete Kurve stellt das Absorptionsspektrum des Dinitrotoluols in der Toluoldiamin/$H_2O$-Matrix dar. Bei einer Wellenlänge von 500 nm wird wiederum der Hauptanteil des absorbierenden Lichts durch das Dinitrotoluol absorbiert.

Zur Bestimmung einer Eichgeraden wurden die Konzentrationen zwischen 50 und 1000 ppm variiert. Diese Konzentrationen wurden durch Gaschromatographie nachgemessen. Für beide Substanzen, sowohl Aminonitrotoluol als auch Dinitrotoluol wurde bei einer Wellenlänge der eingestellten UV/VIS-Strahlung jeweils eine Steigung der Eichgeraden von ca. 800 ppm pro Absorptionseinheit bestimmt.

Die Absorption der Toluoldiamin/$H_2O$-Matrix beträgt bei einer Wellenlänge von 500 nm ca. 0,1 (+/-0,05). Daraus ergibt sich eine Nachweisgrenze von 40 bis 50 ppm. Es sind jedoch noch niedrigere Nachweisgrenzen erreichbar, z.B. durch die Messung der Absorption mit größeren Schichtdicken der Lösung.

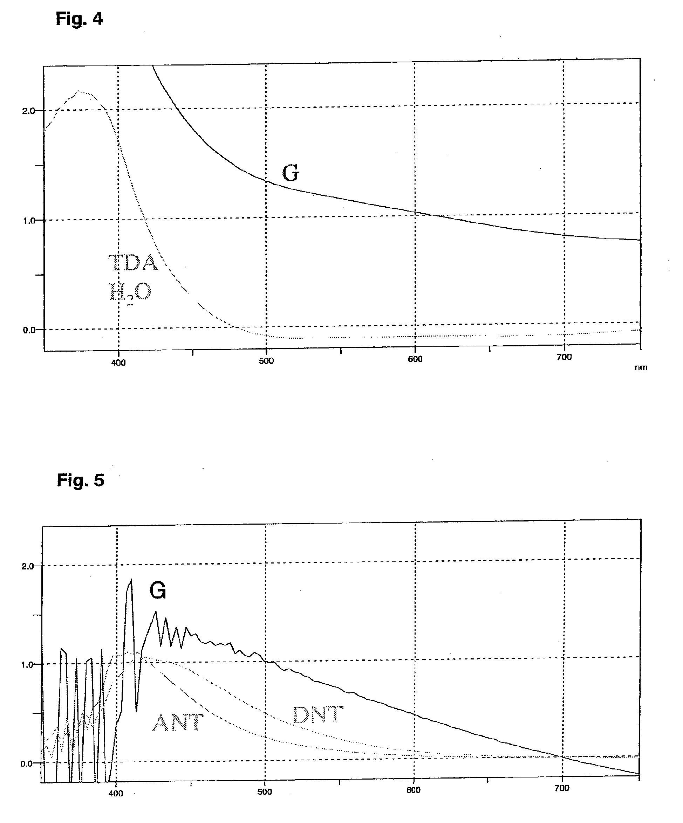

Des Weiteren wurde der Einfluss von Gasblasen auf das Absorptionsmessergebnis überprüft. Bei einer Wasserstoffkonzentration im Reaktor im Bereich der Sättigung können sich Gasblasen aufgrund des Druckabfalls über den Membranfilter kleine Gasblasen bilden. Diese können durch Streuung der UV/VIS-Strahtung die Spektroskopieergebnisse beeinflussen. Dies stellte jedoch nur ein geringfügiges Problem dar. Insbesondere bei einer vertikalen Anordnung der Messzelle, so dass der Reaktionsgemischstrom aufwärts strömte, ergaben sich im Normalbetrieb keine abweichenden Spektren. In der zweiten Messzelle im Bereich unter Umgebungsdruck bildete sich ab und zu eine große Gasblase, insbesondere verursacht durch Änderungen des Betriebszustandes. Daraus ergaben sich Spektren, wie in Figur 4 gezeigt.

Figur 4 zeigt ein UVNIS-Absorptionsspektrum eines Reaktionsgemisches mit Toluoldiamin/$H_2O$-Matrix, das von einer Gasblase beeinflusst wurde. Die Abweichung des mit G bezeichneten "Gasblasen-Spektrums" von den erwarteten Aminonitrotoluol- und Dinitrotoluol-Spektren ist dabei ausreichend groß, um ein solches gemessenes Spektrum (zum Beispiel durch eine entsprechende Software) als nicht für die Konzentrationsbestimmung verwendbar zu identifizieren.

Figur 5 zeigt einen Vergleich von Aminonitrotoluol- und Dinitrotoluol-Absorptionsspektren (ANT und DNT) mit einem mit G bezeichneten, von einer Gasblase verfälschten Absorptionsspektrum. Die Spektren lassen sich deutlich unterscheiden und das "Gasblasen-Spektrum" somit eindeutig erkennen.

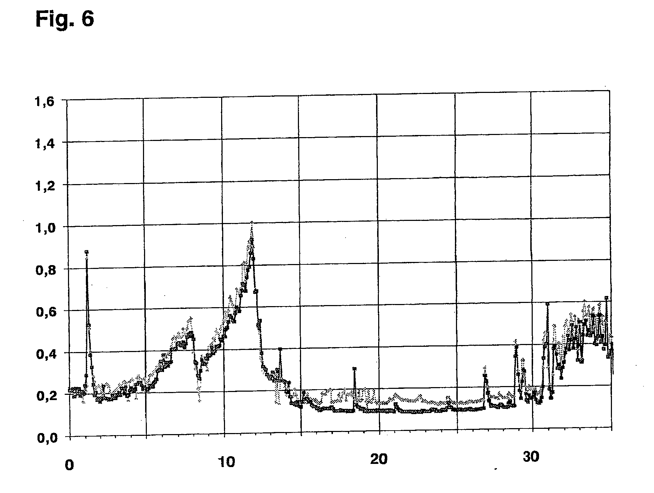

Figur 6 zeigt die Konzentrationsüberwachung des oben erwähnten Pilotreaktors bei der Hydrierung von Dinitrotoluol durch Messung der Absorption von Strahlung mit einer Wellenlänge von 500 nm durch das Reaktionsgemisch. Auf der Y-Achse ist die Absorption (Extinktion) und auf der X-Achse die Zeit in Stunden aufgetragen. Die erste Messzelle (direkt hinter dem Membranfilter) war dabei in einem Bereich mit einem Druck von 20 bis 25 bar angeordnet, die zweite Messzelle bei Umgebungsdruck. Für beide Zellen ist das gemessene Absorptionssignal (siehe Figur 6) fast identisch. In diesem Reaktor wurde der Katalysator diskontinuierlich zugegeben. Sobald eine Deaktivierung des Katalysators vorliegt (zum Beispiel aufgrund von Teer-Ablagerungen auf der Katalysatoroberfläche), ist eine Zunahme der Absorption bei 500 nm beobachtbar, wobei die Zunahme exponentiell ansteigt (wie zwischen der 10ten und der 12ten Stunde zu beobachten). Es kann dann entweder der Dinitrotoluolzulauf reduziert werden (wie in Stunde 8,5 erfolgt) oder neuer Katalysator zugegeben werden (wie in Stunde 11,5 erfolgt). Figur 6 zeigt, dass das erfindungsgemäße Verfahren eine zuverlässige Online-Überwachung der Hydrierung von Dinitrotoluol zu Toluoldiamin durch UV/VIS-Absorptionsmessungen ermöglicht. Damit kann die Sicherheit auch in großen Industriereaktoren erhöht werden. Ferner kann der Katalysatorverbrauch reduziert und gegebenenfalls auch der Deaktivierungsprozess von Katalysatoren verzögert werden.

## Patentansprüche

**1.** Verfahren zur Herstellung von aromatischen Aminen durch Hydrierung von Nitroaromaten oder von aliphatischen Aminoalkoholen durch Hydrierung von Nitroalkoholen in Gegenwart von Katalysatoren, wobei in einem Reaktor ein fluides, Amine oder Aminoalkohole enthaltendes Reaktionsgemisch entsteht, aus dem die Katalysatoren abgetrennt werden, **dadurch gekennzeichnet, dass** nach dem Abtrennen der Katalysatoren eine Messung der Absorption von UV/VIS-Strahlung durch das Reaktionsgemisch zur Konzentrationsbestimmung von Nitro- und Nitrosoverbindungen in dem Reaktionsgemisch durchgeführt wird.

2. Verfahren gemäß Anspruch 1, **dadurch gekennzeichnet, dass** zur Konzentrationsbestimmung der Nitro- und Nitrosoverbindungen ein Absorptionsspektrum in einem Wellenlängenbereich der UV/VIS-Strahtung oder die Absorption von UVNIS-Strahlung mit einer Wellenlänge gemessen wird.

3. Verfahren gemäß Anspruch 2, **dadurch gekennzeichnet, dass** zusätzlich eine Messung der Absorption von UV/VIS-Strahlung durch das Reaktionsgemisch in einem weiteren Wellenlängenbereich oder bei einer weiteren Wellenlänge zur Nulllinienkorrektur durchgeführt wird.

4. Verfahren gemäß einem der Ansprüche 1 bis 3, **dadurch gekennzeichnet, dass** die Katalysatoren aus dem Reaktionsgemisch mit mindestens einem Abtrennverfahren abgetrennt werden, ausgewählt aus der Gruppe Membranfiltration, Sedimentation und Zentrifugation.

5. Verfahren gemäß einem der Ansprüche 1 bis 4, **dadurch gekennzeichnet, dass** die Messung der Absorption von UV/VIS-Strahlung bei einem gegenüber dem Umgebungsdruck erhöhten Druck oder bei Umgebungsdruck durchgeführt wird.

6. Verfahren gemäß einem der Ansprüche 1 bis 5, **dadurch gekennzeichnet, dass** in Abhängigkeit von der Konzentrationsbestimmung der Nitro- und Nitrosoverbindungen in dem Reaktionsgemisch eine dem Reaktor zugeführte Menge an Nitroaromaten oder Katalysatoren geregelt wird.

7. Verfahren gemäß einem der Ansprüche 1 bis 6, **dadurch gekennzeichnet, dass** aromatische Amine durch Hydrierung von Nitroaromaten mit einer oder mehreren Nitrogruppen von 6 bis 18 C-Atomen oder aliphatische Aminoalkohole durch Hydrierung von Nitroalkoholen hergestellt werden.

8. Verfahren gemäß einem der Ansprüche 1 bis 7, **dadurch gekennzeichnet, dass** Toluoldiamin durch Hydrierung von Dinitrotoluol hergestellt wird und eine Messung der Absorption von UV/VIS-Strahlung zur Konzentrationsbestimmung im Wesentlichen von in dem Reaktionsgemisch enthaltenem Dinitrotoluol und Aminonitrotoluol durchgeführt wird.

9. Verfahren gemäß Anspruch 8, **dadurch gekennzeichnet, dass** die Messung der Absorption bei einer Wellenlänge im Bereich von 450 bis 550 nm durchgeführt wird oder ein Absorptionsspektrum in einem Wellenlängenbereich zwischen 350 und 750 nm aufgenommen wird.

10. Vorrichtung zur Durchführung des Verfahrens zur Herstellung von aromatischen Aminen oder von aliphatischen Aminoalkoholen gemäß einem der Ansprüche 1 bis 9 mit einem Reaktor zur Hydrierung von Nitroaromaten oder von Nitroalkoholen in Gegenwart von Katalysatoren, wobei ein Reaktionsgemisch entsteht, mit einem Abtrennelement zum Abtrennen der Katalysatoren aus dem Reaktionsgemisch und mit einem UV/VIS-Spektrometer zur Messung der Absorption von UV/VIS-Strahlung durch das Reaktionsgemisch zur Konzentrationsbestimmung von Nitro- und Nitrosoverbindungen in dem Reaktionsgemisch, wobei die Vorrichtung eingerichtet ist, um das Ergebnis der aus der gemessenen Absorption bestimmten Konzentration an Nitro- und Nitrosoverbindungen zur Regelung des Amin-Herstellungsverfahrens heranzuziehen oder ein Alarmsignal auszulösen, falls eine bestimmte Nitro- und Nitrosoverbindungskonzentration überschritten wird,
wobei die Abtrennung der Katalysatoren aus dem Reaktionsgemisch erfolgt mittels Membranfiltration.

## Claims

1. A process for the preparation of aromatic amines by hydrogenation of nitroaromatics or of aliphatic amino alcohols by hydrogenation of nitro alcohols in the presence of catalysts, in which a fluid reaction mixture which comprises amines or amino alcohols and from which the catalysts are separated off is formed in a reactor, wherein, after the catalysts have been separated off, a measurement of the absorption of UV/VIS radiation by the reaction mixture is carried out to determine the concentration of nitro and nitroso compounds in the reaction mixture.

2. The process according to claim 1, wherein an absorption spectrum is measured in a wavelength range of the UV/VIS radiation or the absorption of UV/VIS radiation having a single wavelength is measured to determine the concentration of the nitro and nitroso compounds.

3. The process according to claim 2, wherein a measurement of the absorption of UV/VIS radiation by the reaction mixture is additionally carried out in a further wavelength range or at a further wavelength for the purposes of baseline correction.

**4.** The process according to any of claims 1 to 3, wherein the catalysts are separated off from the reaction mixture by means of at least one separation process selected from the group consisting of membrane filtration, sedimentation and centrifugation.

**5.** The process according to any of claims 1 to 4, wherein the measurement of the absorption of UV/VIS radiation is carried out at a pressure above ambient pressure or at ambient pressure.

**6.** The process according to any of claims 1 to 5, wherein an amount of nitroaromatics or catalysts fed to the reactor is regulated as a function of the determination of the concentration of the nitro and nitroso compounds in the reaction mixture.

**7.** The process according to any of claims 1 to 6, wherein aromatic amines are prepared by hydrogenation of nitroaromatics having one or more nitro groups and from 6 to 18 carbon atoms or aliphatic amino alcohols are prepared by hydrogenation of nitro alcohols.

**8.** The process according to any of claims 1 to 7, wherein toluenediamine is prepared by hydrogenation of dinitrotoluene and a measurement of the absorption of UV/VIS radiation is carried out to determine the concentration of essentially dinitrotoluene and aminonitrotoluene comprised in the reaction mixture.

**9.** The process according to claim 8, wherein the measurement of the absorption is carried out at a wavelength in the range from 450 to 550 nm or an absorption spectrum is recorded in a wavelength range from 350 to 750 nm.

**10.** An apparatus for carrying out the process for preparing aromatic amines or aliphatic amino alcohols according to any of claims 1 to 9, which comprises a reactor for the hydrogenation of nitroaromatics or of nitro alcohols in the presence of catalysts to form a reaction mixture, and a separation element for separating off the catalysts from the reaction mixture and a UV/VIS spectrometer for measuring the absorption of UV/VIS radiation by the reaction mixture to determine the concentration of nitro and nitroso compounds in the reaction mixture, where the apparatus is set up to employ the result of the concentration of nitro and nitroso compounds determined from the measured absorption for regulating the amine preparation process or to trigger an alarm signal if a particular nitro and nitroso compound concentration is exceeded, and where the catalysts are removed from the reaction mixture by means of membrane filtration.

## Revendications

**1.** Procédé de fabrication d'amines aromatiques par hydrogénation de composés nitroaromatiques ou d'aminoalcools aliphatiques par hydrogénation de nitroalcools en présence de catalyseurs, un mélange réactionnel fluide contenant des amines ou des aminoalcools se formant dans un réacteur, à partir duquel les catalyseurs sont séparés, **caractérisé en ce qu'**après la séparation des catalyseurs, une mesure de l'absorption de rayonnement UV/VIS par le mélange réactionnel est réalisée pour déterminer la concentration en composés nitro et nitroso dans le mélange réactionnel.

**2.** Procédé selon la revendication 1, **caractérisé en ce qu'**un spectre d'absorption dans une plage de longueurs d'onde du rayonnement UV/VIS ou l'absorption d'un rayonnement UV/VIS d'une longueur d'onde est mesuré pour déterminer la concentration en composés nitro et nitroso.

**3.** Procédé selon la revendication 2, **caractérisé en ce qu'**une mesure de l'absorption de rayonnement UV/VIS par le mélange réactionnel dans une autre plage de longueurs d'onde ou à une autre longueur d'onde est également réalisée pour la correction de la ligne de base.

**4.** Procédé selon l'une quelconque des revendications 1 à 3, **caractérisé en ce que** les catalyseurs sont séparés du mélange réactionnel par au moins un procédé de séparation choisi dans le groupe constitué par la filtration sur membrane, la sédimentation et la centrifugation.

**5.** Procédé selon l'une quelconque des revendications 1 à 4, **caractérisé en ce que** la mesure de l'absorption de rayonnement UV/VIS est réalisée à une pression élevée par rapport à la pression ambiante ou à la pression ambiante.

**6.** Procédé selon l'une quelconque des revendications 1 à 5, **caractérisé en ce qu'**une quantité de composés nitroaromatiques ou de catalyseurs introduite dans le réacteur est ajustée en fonction de la détermination de la concentration en composés nitro ou nitroso dans le mélange réactionnel.

**7.** Procédé selon l'une quelconque des revendications 1 à 6, **caractérisé en ce que** des amines aromatiques sont fabriquées par hydrogénation de composés nitroaromatiques contenant un ou plusieurs groupes nitro de 6 à 18 atomes C ou des aminoalcools aliphatiques par hydrogénation de nitroalcools.

**8.** Procédé selon l'une quelconque des revendications 1 à 7, **caractérisé en ce que** de la toluènediamine est fabriquée par hydrogénation de dinitrotoluène et une mesure de l'absorption de rayonnement UV/VIS est réalisée principalement pour déterminer la concentration en dinitrotoluène et d'aminonitrotoluène contenus dans le mélange réactionnel.

**9.** Procédé selon la revendication 8, **caractérisé en ce que** la mesure de l'absorption est réalisée à une longueur d'onde dans la plage allant de 450 à 550 nm ou un spectre d'absorption est enregistré dans une plage de longueurs d'onde comprise entre 350 et 750 nm.

**10.** Dispositif pour la réalisation du procédé de fabrication d'amines aromatiques ou d'aminoalcools aliphatiques selon l'une quelconque des revendications 1 à 9, comprenant un réacteur pour l'hydrogénation de composés nitroaromatiques ou de nitroalcools en présence de catalyseurs, dans lequel un mélange réactionnel se forme, comprenant un élément de séparation pour la séparation des catalyseurs du mélange réactionnel et comprenant un spectromètre UV/VIS pour la mesure de l'absorption de rayonnement UV/VIS par le mélange réactionnel pour déterminer la concentration en composés nitro et nitroso dans le mélange réactionnel, le dispositif étant conçu pour utiliser le résultat de la concentration en composés nitro et nitroso déterminée à partir de l'absorption mesurée pour le réglage du procédé de fabrication d'amines ou pour déclencher un signal d'alarme si une concentration en composés nitro et nitroso déterminée est dépassée, la séparation des catalyseurs du mélange réactionnel ayant lieu par filtration sur membrane.

Fig. 1

2.0
1.0
0.0
400
500
600
700
nm

Fig. 2

2.0
1.0
0.0
400
500
600
700
nm
ANT
TDA
$H_2O$

Fig. 3
2.0
1.0
0.0
DNT
TDA
H2O
400
500
600
700
nm

Fig. 4

2.0
1.0
0.0
400
500
600
700
nm
G
TDA
$H_2O$

Fig. 5

2.0
1.0
0.0
400
500
600
700
G
ANT
DNT

Fig. 6

1,6
1,4
1,2
1,0
0,8
0,6
0,4
0,2
0,0
0
10
20
30

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE OS2044657 A
- EP 1138665 B1
- EP 0978505 B1
- EP 634391 A
- WO 0035852 A
- WO 03066571 A1
- WO 02088860 A2
- DE 3245318 A1
- DE 3040631 A1
- WO 03066571 A
- EP 1310302 A2
- WO 03068724 A